# EUROPEAN PATENT SPECIFICATION

(11) **EP 4 135 646 B1**
(45) Date of publication and mention of the grant of the patent: **29.04.2026**
(21) Application number: 21789406.2
(22) Date of filing: 24.03.2021
(51) Int. Cl.: A61J 1/20, A61J 1/14, A61M 5/24, A61M 5/28, A61M 5/31, A61M 5/32, A61M 5/34

(54) **NEEDLE CONNECTORS AND MODULAR NEEDLE CONNECTORS FOR MULTI-DOSE DRUG DELIVERY DEVICES AND METHODS THEREOF**
NADELVERBINDER UND MODULARE NADELVERBINDER FÜR VORRICHTUNGEN ZUR ABGABE VON MEDIKAMENTEN MIT MEHREREN DOSEN UND VERFAHREN DAFÜR
CONNECTEURS D'AIGUILLE ET CONNECTEURS D'AIGUILLE MODULAIRES POUR DISPOSITIFS D'ADMINISTRATION DE MÉDICAMENT MULTI-DOSE ET PROCÉDÉS ASSOCIÉS

(30) Priority: 16.04.2020 US 202063011029 P; 01.09.2020 US 202063073158 P
(43) Date of publication of application: 22.02.2023
(73) Proprietor: Takeda Pharmaceutical Company Limited, Osaka-shi, Osaka 541-0045 (JP)
(72) Inventor: PIRBODAGHI, Tohid, Lexington, Massachusetts 02421 (US)
(74) Representative: Graham Watt & Co
(86) International application number: PCT/US2021/023888
(87) International publication number: WO 2021/211273

(56) References cited:
- WO-A1-93/25251
- US-A- 5 829 589
- US-A1- 2010 036 362
- US-A1- 2019 125 959
- US-B2- 9 180 070
- US-B2- 9 180 070

## Description

### Cross References to Related Applications

This Application claims priority, and benefit under 35 U.S.C. § 119(e), to U.S. Provisional Patent Application No. 63/011,029, filed 16 April 2020, and to U.S. Provisional Patent Application No. 63/073,158, filed 01 September 2020.

### Field of the Disclosure

This invention generally relates to multi-dose injection systems for delivering drugs, hormones, biologics, and other therapeutic agents. More particularly, this invention relates to needle connectors having varied proximal needle locations corresponding to varied piercing locations on a septum of a reusable injector.

### Background

Many medical conditions exist that require periodic injections to treat, alleviate, or prevent the condition's underlying symptoms. Consider for example a diabetic that requires periodic doses of insulin, or a patient with a thyroid disorder requiring daily injections of a hormone. One commonality between all periodic, multi-dose injection regimens is that any disruption in the administration of the drug, hormone, biologic, or other therapeutic agent can cause significant problems for the patient. This can include discomfort, pain, relapse of a condition, or possibly even death.

One current method of providing multi-dose, periodic injection regimens is to provide a reusable injector that includes the therapeutic agent. A single injector can be reused over several injection regimens (e.g., hourly, weekly, bi-weekly, etc.). To ensure a sterile injection device, the injection system can include multiple single-use needle connectors that attach to the tip of the reusable injector. The system can include a quantity of needle connectors that corresponds to the quantity of injections provided by the reusable injector (e.g., 14 needle connectors for a 14-day injection regimen).

The needle connectors can have a needle with a proximal end and a distal end. The distal end can be inserted into the patient, for example subcutaneously, to administer the therapeutic agent. The proximal end can be inserted into the reusable injector, where the tip of the proximal end pierces a septum to access to the therapeutic agent housed within the injector.

A problem exists with existing injection systems as it relates to the method of piercing the septum of the reusable injector. Previous needle connectors all pierced the septum in the same location. When the expectation is that numerous needle connectors are used with the same reusable injector, for example 14 needles for a 14-day regimen, piercing and re-piercing the septum can cause trauma to the septum. This trauma can lead to the generation of rubber particles that sheer off into the therapeutic agent within the reusable injector. This could lead to the contamination of the therapeutic agent and, potentially, adverse effects to the patient.

There is therefore a need for improved methods, devices, and systems to provide to multi-dose injection regimens without risking contamination of the therapeutic agent disposed within the reusable injector.

US 9 180 070 B2 discloses an adaptor for a vial which includes an opening to select and align with one of several holes and pierce an elastomeric piece in different locations.

### Summary

It is an object of the present invention to provide systems, devices, and methods to meet the above-stated needs. Generally, it is an object of the present invention to provide an injection system with needle connectors having varied proximal needle locations corresponding to varied piecing locations on the septum of a reusable injector.

An example system described herein can include a reusable injector having a septum. The system can include a plurality of attachable needle connectors sized to attach to a distal end of the reusable injector proximate the septum. The plurality of attachable needle connectors can include a first needle connector having a first needle. A proximal end of the first needle can correspond to a first location on the septum. The plurality of attachable needle connectors can include a second needle connector having a second needle. A proximal end of the second needle can correspond to a second location on the septum. The first location and the second location are different locations such that the septum can be pierced at different locations by the first needle connector and the second needle connector.

The system can include a first set of needle connectors. The first set can include the first needle connector and a third needle connector having a third needle. A proximal end of the third needle can correspond to the first location on the septum, similar to the first needle connector.

The distal end of the reusable injector can have a circular cross section. A proximal end of each of the plurality of attachable needle connectors can be concentrically positionable upon the distal end of the reusable injector. The proximal end of the first needle can be positioned concentric with the distal end of the reusable injector. The proximal end of the second needle can positioned nonconcentric with the distal end of the reusable injector.

The distal end of the reusable injector can include a first anti-rotation feature. Each of the plurality of attachable needle connectors can include a second anti-rotation feature sized to engage the first anti-rotation feature.

The distal end of the reusable injector can have a circular cross section. A proximal end of each of the plurality of attachable needle connectors can be concentrically positionable upon the distal end of the reusable injector. The proximal end of the first needle can be offset from the second anti-rotation feature at a first radial offset. The proximal end of the second needle can be offset from the second anti-rotation feature at a second radial offset. The first radial offset can be different than the second radial offset such that the septum can be pierced at different locations by the first needle connector and the second needle connector.

The first anti-rotation feature can be one of a keyway or a key, and the second anti-rotation feature can one of a key or a keyway and opposite the first anti-rotation feature.

The first anti-rotation feature can be a first cross-sectional geometric shape of the distal end of the reusable injector. The second anti-rotation feature can be a second cross-sectional geometric shape of a proximal end of each of the plurality of attachable needle connectors. The first cross-sectional geometric shape and the second cross-sectional geometric shape can be non-circular to prevent rotation between the features.

The second needle can have a curve disposed between the proximal end of the second needle and a distal end of the second needle.

An example kit can include a plurality of needle connectors attachable to a reusable injector. The kit can include a first set of one or more needle connectors, each of the needle connectors including a first needle. A proximal end of each first needle can be positioned to puncture a first location on a septum of the reusable injector. A second set one or more of needle connectors, each of the needle connectors having a second needle. A proximal end of each second needle can be positioned to puncture a second location on the septum. The first location and the second location are different locations such that the septum can be pierced at different locations by the set and the second set.

The first set and/or the second set can include two to five needle connectors. In some examples, the first set and/or the second set can be limited to no more than five needle connectors.

The first set can include a first needle connector and a second needle connector. The first needle connector can be indexed for a first injection regimen, and the second needle connector can be indexed for a second injection regimen. The second set can include a third needle connector and a fourth needle connector. The third needle connector can be indexed for a third injection regimen, and the fourth needle connector can be indexed for a fourth injection regimen.

The first set can include the said first needle connector, and the second set can include the said second needle connector. The kit can further include a third needle connector, a fourth needle connector, a fifth needle connector, a sixth needle connector, and a seventh needle connector. Each needle connector can be indexed for one injection regimen of a seven-dose injection regimen. A proximal end of each needle connector can be positioned to puncture a different location on the septum.

Each needle connector of the first set can include a first anti-rotation feature sized to correspond with a second anti-rotation feature on the reusable injector. Each needle connector of the second set can include a third anti-rotation feature can be sized to correspond with the second anti-rotation feature on the reusable injector.

The first anti-rotation feature and the third anti-rotation feature can be one of a keyway or a key, and the second anti-rotation feature can be one of a key or a keyway and opposite the first anti-rotation feature and the third anti-rotation feature.

The first anti-rotation feature and the third anti-rotation feature can be a first cross-sectional geometric shape of a proximal end of each needle connector of the first set and the second set. The second anti-rotation feature can be a second cross-sectional geometric shape of a distal end of the reusable injector. The first cross-sectional geometric shape and the second cross-sectional geometric shape can be non-circular.

An example method can include selecting a first needle connector for a first injection regimen. The first needle connector can be positioned proximate a distal end of a reusable injector. A septum of the reusable injector can be pierced at a first location with a proximal end of a first needle of the first needle connector. The first injection regimen can then be administered to a patient. A second needle connector can be selected for a second injection regimen. The second needle connector can be positioned proximate the distal end of the reusable injector. The septum of the reusable injector can be pierced at a second location with a proximal end of a second needle of the second needle connector. The second injection regimen can then be administered to the patient. The first location and the second location can be different locations on the septum and/or the corresponding needle connector.

The method can include rotating the first needle connector to pair a first anti-rotation feature upon the first needle connector with a second anti-rotation feature upon the reusable injector.

The method can include selecting a third needle connector for a third injection regimen. The third needle connector can be positioned proximate the distal end of the reusable injector. The septum can be pierced at the first location on the septum with a proximal end of a third needle of the third needle connector. The third injection regimen can then be administered to the patient.

An example modular needle connector can include a circular disk having a needle aperture positioned non-concentrically with a center of the circular disk. The modular needle connector can include a needle positioned within the needle aperture. The needle can have a distal end extending from a first side of the circular disk and a proximal end extending from a second side of the circular disk. The modular needle connector can further include a connector body. The connector body can include a disk slot sized to contain the circular disk and positioned at a first end of the connector body. The connector body can include an injector housing positioned at a second end of the connector body and sized to engage a reusable injector. The proximal end of the needle can be positioned within the injector housing and the circular disk is rotatable within the connector body.

A first height and first diameter of the circular disk can be less than a second height and a second diameter of the disk slot such that the circular disk is rotatable within the disk slot when the connector body is disengaged with the reusable injector.

The connector body can include a plurality of flexible wings extending along at least a portion of a length the injector housing. An interior surface of the plurality of flexible wings can include threads sized to engage the reusable injector.

The distal end of the needle can include a bevel-tip and the proximal end of the needle can include a non-coring tip. The non-coring tip can include a blunt tip and a side-mounted fluid inlet.

A system according to the present disclosure can include a reusable injector including a septum. The system can further include a modular needle connector. The module needle connector can include a rotatable disk including a needle extending therethrough at a position non-concentric with a center of the rotatable disk. The needle can include a distal end extending from a first side of the rotatable disk and a proximal end extending from a second side of the rotatable disk. The module needle connector can include a connector body. The connector body can include a disk slot sized to contain the rotatable disk and positioned at a first end of the connector body. The connector body can include an injector housing positioned at a second end of the connector body and sized to engage the reusable injector. The proximal end of the needle can extend into the injector housing and can be movable from a first position within the injector housing to a second position within the injector housing via rotation of the rotatable disk.

The connector body can include a plurality of flexible wings sized to engage the reusable injector proximate the septum. An interior surface of the plurality of flexible wings can include first threads sized to engage second threads on the reusable injector proximate the septum.

An interior surface of the injector housing can include first threads sized to engage second threads on the reusable injector proximate the septum. The distal end of the needle can include a bevel-tip, and the proximal end of the needle can include a non-coring tip. The non-coring tip can include a blunt tip and a side-mounted fluid inlet.

A first axis of the rotatable disk can be co-axial with a second axis of the septum. The needle can be off-axis from the first axis and the second axis.

The proximal end of the needle can be stationary when inserted into the septum. The rotatable disk can be stationary when the connector body is seated onto the reusable injector.

A method can include positioning a modular needle connector proximate a distal end of an injector. The method can include piercing, with a proximal end of a needle within the modular needle connector, a septum of the injector at a first location. The needle can be connected to a rotatable disk within the modular needle connector. The method can include advancing the modular needle connector axially onto the distal end of the injector. The method can include rotating a connector body of the modular needle connector to disengage first threads on the connector body from second threads on the distal end of the injector. Rotating the connector body can cause the rotatable disk to rotate as the proximal end of the needle is withdrawn axially from the first location. The method can include removing the modular needle connector from the distal end of the injector.

The method can include piercing, with the proximal end of the needle, the septum at a second location. The method can include rotating the connector body to disengage the first threads from the second threads on the distal end of the injector. Rotating the connector body can cause the rotatable disk to rotate as the proximal end of the needle is withdrawn axially from the second location.

The needle can be positioned non-concentrically with a center of the rotatable disk.

Advancing the modular needle connector axially onto the distal end of the injector can include advancing flexible wings over the distal end of the injector. The first threads can be positioned on an interior surface of the flexible wings.

The method can include administering an injection regimen via a distal end of the needle.

### Brief Description of the Drawings

The above and further aspects of this invention are further discussed with reference to the following description in conjunction with the accompanying drawings, in which like numerals indicate like structural elements and features in various figures. The drawings are not necessarily to scale, emphasis instead being placed upon illustrating principles of the invention. The figures depict one or more implementations of the inventive devices, by way of example only, not by way of limitation.
FIG. 1 is a side-view illustration of a needle connector assembly, according to aspects of the present disclosure;
FIGs. 2A-2C are side-view illustrations of needle connectors, according to aspects of the present disclosure;
FIGs. 3A and 3B are perspective views of needle connectors and corresponding reusable injectors, according to aspects of the present disclosure;
FIGs. 4A-4C are side views of an example method of attaching a needle connector to a reusable injector, according to aspects of the present disclosure;
FIGs. 5A-5B are end views of example needle placements for needle connectors, according to aspects of the present disclosure;
FIGs. 6A-6B illustrate another example needle placement for needle connectors, according to aspects of the present disclosure;
FIG. 7 illustrates yet another example of a needle placement pattern, according to aspects of the present disclosure;
FIG. 8 illustrates a further example of a needle placement pattern, according to aspects of the present disclosure;
FIG. 9 illustrates an example of a needle placement pattern with an anti-rotation feature, according to aspects of the present disclosure;
FIG. 10 illustrates an example of a needle placement pattern with another example of an anti-rotation feature, according to aspects of the present disclosure;
FIG. 11 illustrates an example of a needle placement pattern with a further example of an anti-rotation feature, according to aspects of the present disclosure;
FIG. 12 is a flow diagram illustrating a method of attaching needle connectors to a reusable injector, according to aspects of the present disclosure;
FIG. 13 is a perspective view of a modular needle connector, according to aspects of the present disclosure;
FIGs. 14A and 14B depict and example disk for a modular needle connector, according to aspects of the present disclosure;
FIGs. 15A and 15B depict and example connector body for a modular needle connector, according to aspects of the present disclosure;
FIGs. 16A and 16B are side sectional views of an assembled modular needle connector, according to aspects of the present disclosure;
FIGs. 17A and 17B are side views of an example method of attaching a modular needle connector to a reusable injector, according to aspects of the present disclosure; and
FIG. 18 is a flow diagram illustrating a method of attaching and removing a modular needle.

### Detailed Description

The herein disclosed solution is directed to multi-dose injection systems for delivering drugs, hormones, biologics, and other therapeutic agents. In particular, the solution describes modular, attachable needle connectors that pierce the septum of reusable injectors at varying locations. As was described above, current methods for administering multi-dose injection regimens mostly include a reusable injector containing the therapeutic injection and a multiple needle connectors that can be attached to the distal end of the injector for each injection. The reusable injector can have several doses of therapeutic agent, and thus the system can include as many needles as required for the full regimen. For example, it is common for a system to provide seven needle connectors for a week-long regimen, fourteen needle connectors for a two-week-long regimen, and so forth.

One issue with current systems is the repeated trauma administered to the same location on the septum of the reusable injector. It has been shown that piercing/puncturing the same location of a septum repeatedly can cause deterioration of the rubber-material of the septum. This, in turn, can cause the therapeutic agent disposed within the reusable injector to become contaminated by rubber fragments of the deteriorated septum. This is, of course, not ideal for a system that contains a drug, hormone, biologic, or other agent that is to be injected into the patient.

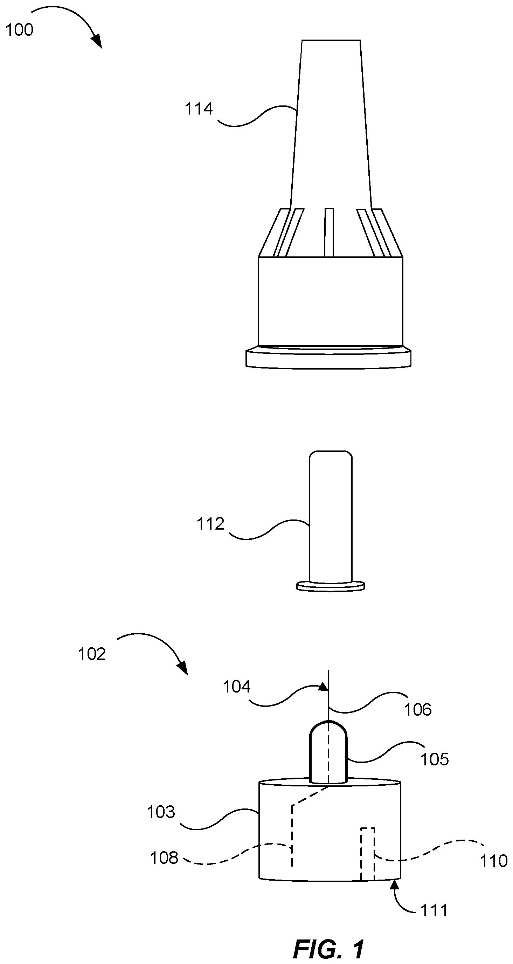

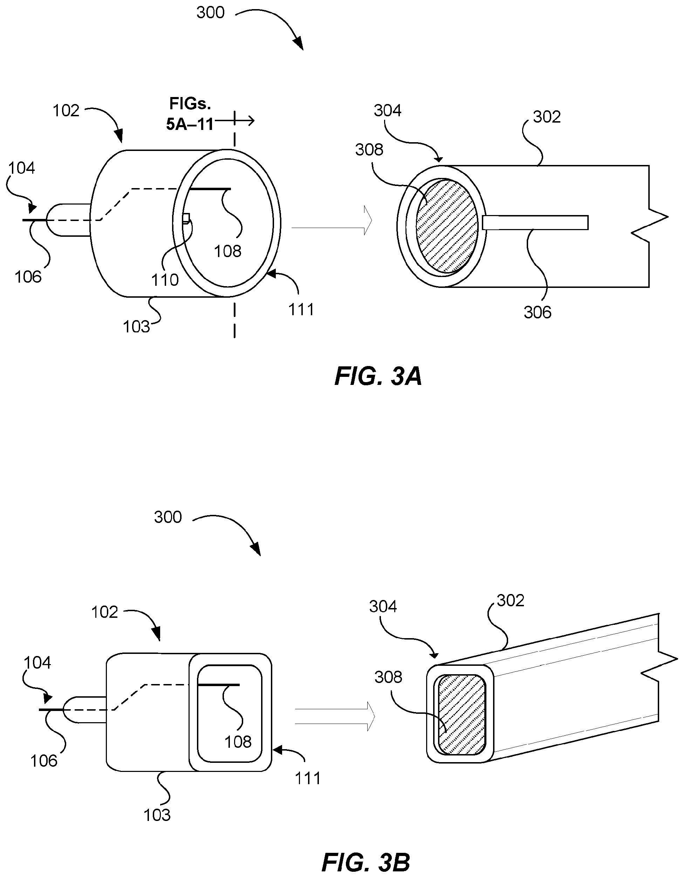

The devices, systems, and methods described herein provide solutions to this contamination problem by limiting the number of times a septum is pierced at any given location. Various devices and methods are disclosed for providing an injection system with multiple needle connectors, and examples of the devices and methods will now be described with reference to the accompanying figures. FIG. 1 is a side-view illustration of a needle connector assembly 100, according to aspects of the present disclosure. The needle connector assembly 100 can include a needle connector 102. The needle connector 102 can have a casing 103 that can both secure the needle connector 102 to a reusable injector (e.g., the reusable injector 302 shown in FIGs. 3A and 3B) and protect a proximal end 108 of a needle 104.

The needle connector 102 can include a needle 104 that can be used to both pierce the septum 308 of the reusable injector 302 and puncture the skin of a patient for delivering the therapeutic agent. For example, the needle 104 can have a distal end 106 extending from the casing 103 or a protrusion 105 that also extends from the casing 103. The distal end 106 of the needle 104 can be used to puncture the skin of the patient and deliver the therapeutic agent, for example subcutaneously. A needle connector 102 can include the aforementioned protrusion 105 to provide lateral stability to the distal end 106 of the needle 104 so that the end of the needle 104 being inserted into the patient can be more rigid. The needle 104 can have a proximal end 108 that extends into the casing 103 and, as will be described in greater detail below, pierces the septum 308 of the reusable injector 302.

The needle connector 102 can also include an anti-rotation feature 110 that can ensure the proximal end 111 of the needle connector 102 is positioned properly upon the reusable injector 302. In previous systems, the reusable injectors and the needle connectors commonly had a circular cross-section. The needles of the previous needle connectors were positioned centrally (e.g., concentrically) in the outer casing of the needle connector and were intended to pierce the center of the circular septum. Because of this, the needle connector could be rotated and attached to the injector at any position, as the needle pierced the septum at the same location regardless of rotation. With the current systems and methods, the proximal end 108 of the needles 104 can be located at various positions. To this end, the anti-rotation feature 110 of the needle connector 102, along with a corresponding anti-rotation feature of the reusable injector 302 (e.g., anti-rotation feature 306 shown in FIGs. 3A and 3B), can ensure the piercing location on the septum 308 is the intended piercing location. The anti-rotation feature 110 is discussed in greater detail below.

The needle connector assembly 100 can include a needle protector 112. The needle protector 112 can be a removable enclosure of the distal end 106 of the needle 104 extending from the needle connector 102. The needle protector 112 can sized to engage, for example, the protrusion 105. The needle protector 112 can provide a degree of safety to the patient by ensuring that the needle 104 is not exposed prior to the patient needing access to the needle 104.

The needle connector assembly 100 can include a connector cap 114. The connector cap 114 can be used to protect the needle connector 102 and needle protector 112 during transport and handling. The connector cap 114 can also be used to ensure sterility of the needle connector 102. For example, the connector cap 114 can be sized to extend over and be placed upon the casing 103 so that the distal end 106 of the needle and casing 103 can be enclosed. A seal (made of, for example, paper, plastic, foil, etc.) can be positioned upon the end of the connector cap 114 proximate the proximal end 111 of the needle connector 102 so that the entire needle connector 102 can be sealed for sterile storage.

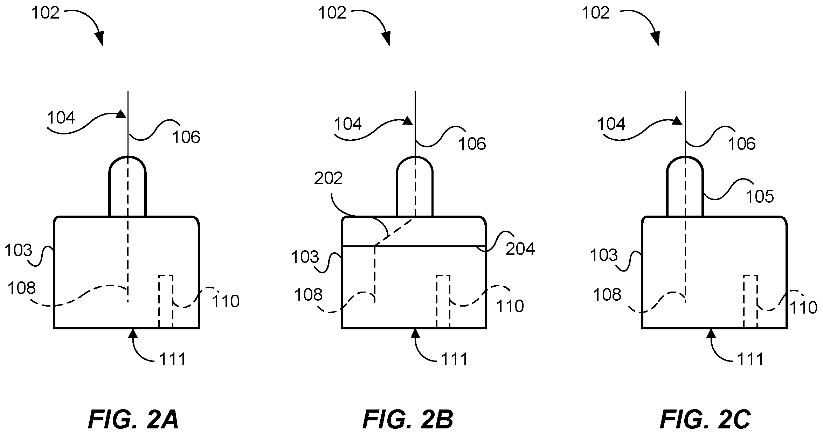

FIGs. 2A-2C are side-view illustrations of needle connectors 102, according to aspects of the present disclosure. The examples shown in FIGs. 2A-2C illustrate different placements and/or shapes of a needle 104 in corresponding needle connectors 102. As described above, the casing 103 of each needle connector 102 can be sized to engage the reusable injector 302 (e.g., at a distal end 304 of the reusable injector 302as shown in FIGs. 3A and 3B). As shown in FIG. 2A, the needle 104 can be placed centrally within the casing 103 and extend straight. This design causes the distal end 106 of the needle 104 to be positioned centrally within the needle connector 102 and the proximal end 108 of the needle 104 to be positioned, correspondingly, centrally (e.g., concentrically) with respect to distal end 304 of the reusable injector 302.

Alternatively, the proximal end 108 of the needle 104 can be positioned at other locations within the casing 103 such that the proximal end 108 of the needle 104 corresponds to non-centrally located positions on the distal end 304 of the reusable injector 302. In the example shown in FIG. 2B, this is accomplished by providing a curve 202 in the needle 104 disposed between the proximal end 108 and distal end 106 of the needle 104. The curve 202 can be an offset, as shown in the figure, or a bend in the needle. This design causes the distal end 106 of the needle 104 to be positioned centrally within the needle connector 102, but the proximal end 108 is positioned non-centrally within the casing 103. This can cause the proximal end 108 of the needle 104 shown in FIG. 2B to puncture a different location on the septum 308 than the proximal end 108 of the needle 104 shown in FIG. 2A.

The curve 202 in the needle 104 can be secured within the casing 103, for example, by a curve protection layer 204 disposed within the needle connector 102. The inside of the casing 103 can be generally hollow so as to connect to the distal end 304 of the reusable injector 302. The curve protection layer 204 can be a solid layer in the casing 103 material to encase and protect the curve 202 in the needle 104. This, of course, can prevent the needle 104 from flexing as it is inserted into the septum 308, as the proximal end 108 can extend straight from the curve protection layer 204 and toward the septum 308.

In yet another example, as shown in FIG. 2C, the proximal end 108 can be offset within the casing 103 by offsetting the entire needle 104 while providing a straight needle 104 (i.e., no curve 202 is provided in the design). This design can accomplish the same result for the proximal end 108 of the needle 104, but the distal end 106 of the needle 104 can also be offset with respect to the casing 103. This design can eliminate the need for a curve protection layer 204 as the needle 104 may not be as susceptible to flexing as the needle 104 is inserted into the septum 308.

FIGs. 3A and 3B are perspective views of needle connectors 102 and corresponding reusable injectors 302, according to aspects of the present disclosure. The injection systems 300 described herein comprise at least two components, a reusable injector 302 and needle connectors 102 positionable upon, and attachable to, the reusable injectors 302. Although the injection systems shown in FIGs. 3A and 3B show only a single needle connector 102, the injection system 300 can be multi-use, such that the injection system 300 includes a plurality of needle connectors 102. The proximal end 111 of the needle connector 102 can have a cross-sectional geometry that corresponds to the cross-sectional geometry of the distal end 304 of the reusable injector 302 so that the two are connectable by sliding the needle connector 102 axially upon the reusable injector 302.

Also note that examples of the needle connector 102 can be threadably engaged to the reusable injector 302. As the needle connector 102 is threaded down, the threads can be such to provide the proper location. An alternate example has the anti-rotation feature 110 on the needle connector 102 engage the anti-rotation feature 306 on the reusable injector 302 at the point of the final tightening or course of thread to again sit the proximal end 108 of the needle 104 in the designated location on the septum 308.

The perspective views of FIGs. 3A and 3B provide an unobstructed view of how the proximal end 108 of each needle 104 can be inserted into a septum 308 of the reusable injector 302. As can be seen in both figures, the proximal end 108 of the needles 104 can be offset from the center of the septum 308, similar to the examples shown in FIGs. 2B and 2C. Alternatively, the proximal end 108 of the needles 104 can be positioned to correspond to a center location of the septum 308, similar to the example shown in FIG. 2A.

In some examples, the tip of the proximal end 108 of the needle 104 can be slightly recessed into the casing 103. Stated otherwise, the tip of the proximal end 108 of the needle 104 may not extend entirely to the proximal end 111 of the needle connector 102. This can provide a level of safety so that a user does not inadvertently puncture themselves with the needle 104. A recessed needle 104 can also provide a degree of tolerance as the needle connector 102 is slid onto the distal end 304 of the reusable injector 302. For example, as the needle connector 102 is being attached to the reusable injector 302, if the proximal end 108 of the needle 104 is slightly recessed, the proximal end 111 of the needle connector 102 can extend over the distal end 304 of the reusable injector 302 to a greater degree before the proximal end 108 of the needle 104 engages the septum 308. This can also facilitate the engaging of the anti-rotation features 110/306 described herein.

The proximal end 108 of the needle 104 can also be manufactured to further facilitate the protection of the septum 308. For example, the tip of the proximal end 108 of the needle 104 can be a non-coring design so as to prevent rubber particle generation as the septum 308 is pierced.

Referring to FIG. 3A, the distal end 304 of the reusable injector 302 can have a circular cross section, which can match to a proximal end 111 of a needle connector 102 that also has a circular cross section. In these examples, the proximal end 111 of the needle connector 102 can be positioned concentrically upon the distal end 304 of the reusable injector 302. The needle connector 102 and/or the reusable injector 302 can include anti-rotation features to ensure the two are aligned properly. When the proximal end 108 of the needle 104 is to be placed at various locations such that two needle connectors 102 can pierce the septum 308 at different locations, the anti-rotation features can ensure the piercing location on the septum 308 is the intended piercing location.

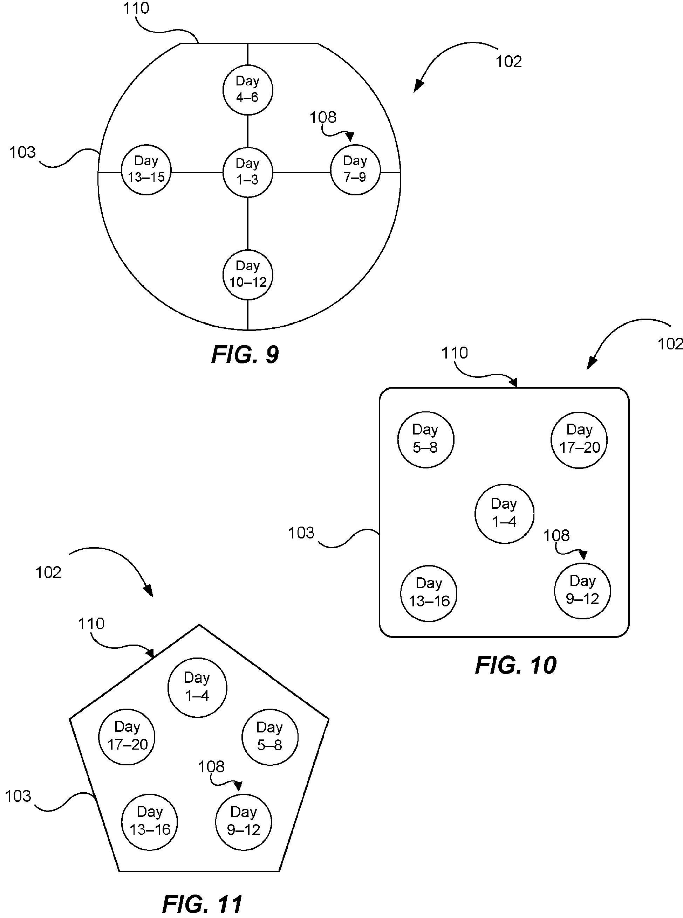

The needle connector 102 can include a first anti-rotation feature 110 and the reusable injector 302 can include a second anti-rotation feature 306. The first anti-rotation feature 110 and the second anti-rotation feature 306 can be a key/keyway pair, as shown in FIG. 3A. For example, the anti-rotation feature 110 of the needle connector 102 can be a key that is sized to engage the anti-rotation feature 306 of the reusable injector 302, which can be a keyway. The reverse, of course, can also be true, wherein the anti-rotation feature 306 of the reusable injector 302 can be a key that engages a keyway of the needle connector 102. Other anti-rotation feature 110/306 are also possible, such as tracks, splines, etc. Another example includes a partial flat space on the otherwise circular cross section of the casing 103 that meets with a flat surface of the otherwise circular cross section of the reusable injector 302 (e.g., the example shown in FIG. 9).

Referring to FIG. 3B, the anti-rotation feature of the needle connector 102 and the anti-rotation feature of the reusable injector 302 can, instead of being a separate feature like a key/keyway, be the cross-sectional geometric shape of the two components. Using the example shown in FIG. 3B, the distal end 304 of the reusable injector 302 can be square or rectangular and the proximal end 111 of the needle connector 102 can be square or rectangular and sized to engage the reusable injector 302. By providing a non-circular cross section, the positions/orientations in which the needle connector 102 can be placed upon the reusable injector 302 can be limited. For example, if both are square, then only four orientations are possible; if both are rectangular, only two orientations are possible. The cross-sectional geometric shape of the two devices can be any other non-circular shape, including but not limited to triangular, trapezoidal, pentagonal, hexagonal, and other cross-sectional geometric shapes.

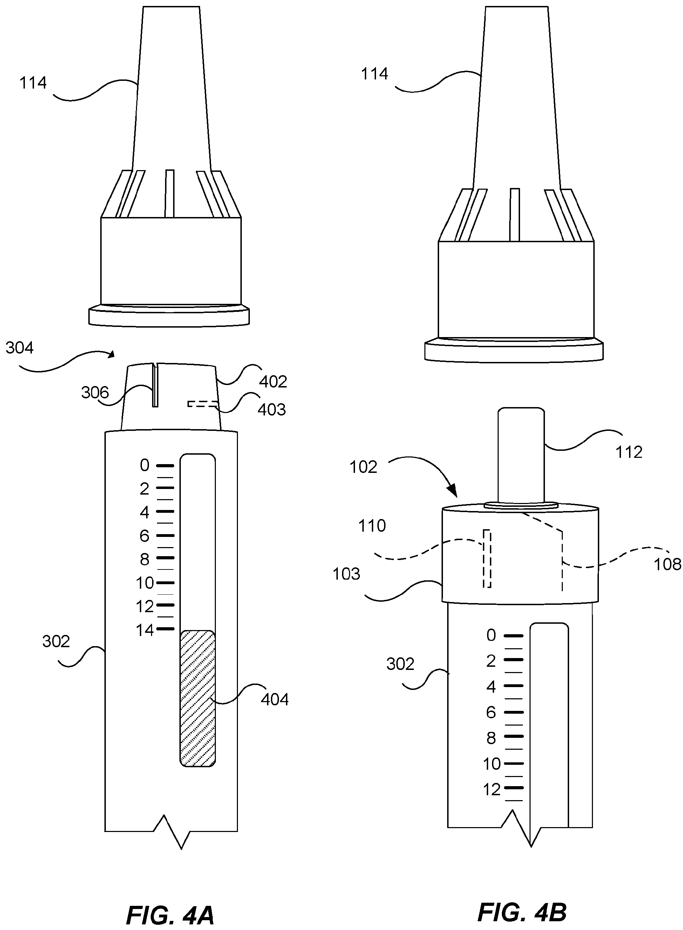

FIGs. 4A-4C are side views of an example method of attaching a needle connector 102 to a reusable injector 302, according to aspects of the present disclosure. As described above, the injection system can include a plurality of needle connectors 102 for each injection regimen and at least one reusable injector 302 for providing a therapeutic agent (e.g., drug, hormone, biologic, etc.). FIGs. 4A-4C show a method of attaching a single needle connector 102 having an offset to the proximal end 108 of the needle 104. As described above, however, multiple needle connectors 102 can be provided to the patient as a kit to cover the entire injection regimen (e.g., one week of daily doses, two weeks of daily doses, or any other periodic injection regimen). Furthermore, although the needle connector 102 in FIGs. 4A-4C has an offset (i.e., non-centrally placed) proximal end 108 of the needle 104, the same process can be used for any needle connector 102 configuration described herein.

In FIG. 4A, a connector cap 114 containing the needle protector 112 and needle connector 102 is placed proximate the distal end 304 of the reusable injector 302. As described above, this step could be preceded by removing a protective seal from the end of the connector cap 114. The connector cap 114, needle protector 112, and needle connector 102 assembly is then slid axially onto the distal end 304 of the reusable injector 302. FIG. 4A shows an example reusable injector 302 having an anti-rotation feature 306 that is a keyway, which is in accordance with the present disclosure. The keyway can be sized to engage a key disposed on the needle connector 102 (i.e., the anti-rotation feature 110 of the needle connector 102). The anti-rotation features 306/110 can also assist in positioning the needle connector 102 as it is pressed axially onto the reusable injector 302, for example by acting as a track to guide the placement of the needle connector 102.

The systems described herein can include a mechanical attachment that removably secures the needle connector 102 to the reusable injector 302. These attachments can ensure the needle connector 102 does not separate from the reusable injector 302 inadvertently, for example as the patient is pulling the needle 104 from their skin. The designs of previous injections systems, as described above, utilized centrally-placed needles that pierce the septum 308 at a central location. Because of this, the systems could, and ordinarily did, include threads on the needle connector 102 and the reusable injector 302 to as to provide the mechanical attachment.

However, other designs can be employed with the presently-described systems and methods. Since the proximal end 108 of the needle 104 can be placed at different, predetermined locations, rotating the needle connector 102 with respect to the reusable injector 302 to engage threads may not be desired and could damage the needle 104. Instead, the distal end 304 of the reusable injector 302 can have a taper 402, for example a Morse taper, that can be sized to engage a corresponding taper on the needle connector 102 (not shown in view of FIG. 4A) to provide the slight mechanical attachment between the two components. In other examples, other mechanical attachments 403 may be applied to one or both of the components, such as a ribs, cantilevers, or other mechanical features for snap fitting the two components together.

The reusable injector 302 can act as a cartridge for housing the therapeutic agent 404. The therapeutic agent 404 can be a drug, hormone, biologic, or other agent that is intended to be injected into a patient via the needle 104 of the needle connector 102. The therapeutic agent 404 can be a liquid or a powder that is reconstituted by a liquid also stored in the reusable injector 302.

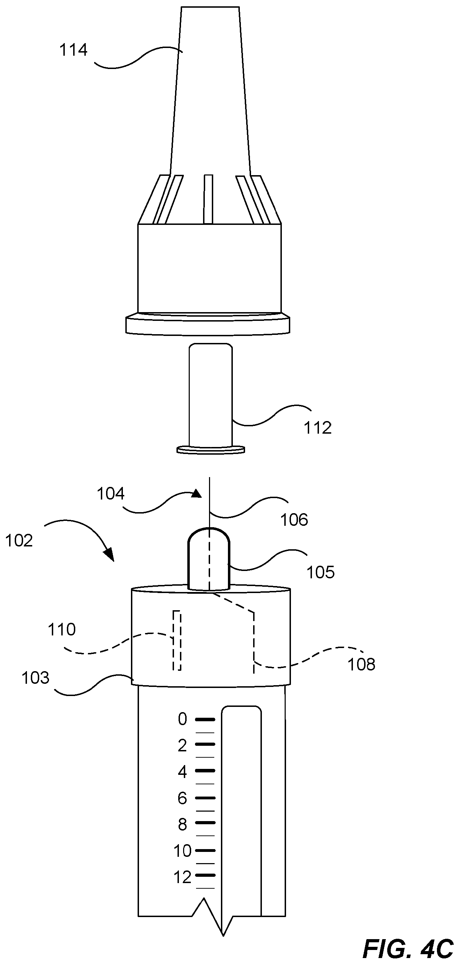

As shown in FIG. 4B, after the needle connector 102 is seated onto the distal end 304 of the reusable injector 302, the connector cap 114 can be removed from the needle connector 102. If the system includes a needle protector 112, the needle protector 112 can now be exposed, and the needle 104 can be covered and protected. As described above, the example system in FIG. 4B includes an offset proximal end 108 of the needle 104. This can enable the proximal end 108 to puncture the septum 308 (not shown in view) at a position off-center. As shown in FIG. 4C, the needle protector 112 can be removed to access the distal end 106 of the needle 104, and the therapeutic agent 404 can be administered to the patient.

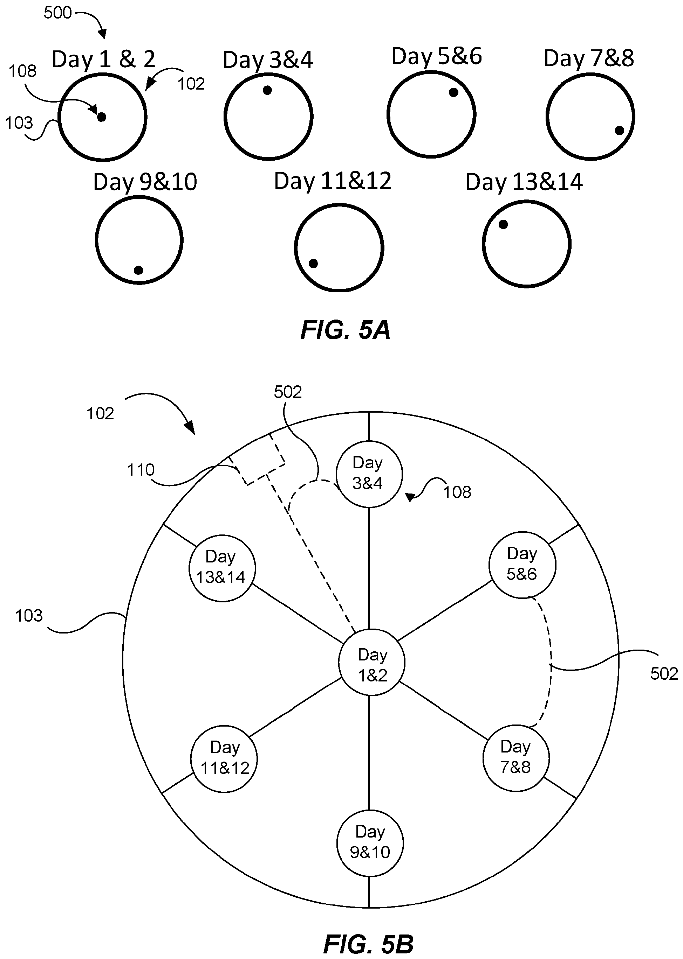

FIGs. 5A-11 are end views of example placements for the proximal end 108 of needles 104 in needle connectors 102, according to aspects of the present disclosure. The views show the relative offsets that can be provided in needle connectors 102, but the examples are not limiting. Since the placement of the proximal end 108 of the needle 104 also corresponds to a piercing location on the septum 308 (not shown in FIGs. 5A-11), the views also indicate the corresponding piercing locations.

FIG. 5A depicts example proximal end 108 needle placement and corresponding indexing 500 for the needle connector 102. Indexing 500 can be provided to let the patient know which needle connector 102 is to be used for the injection regimen. It is contemplated that the plurality of needle connectors 102 can be provided as a kit either alone or in combination with the reusable injector 302. The kit can include all necessary needle connectors 102 to complete the injection regimen for the reusable injector 302. For example, if the regimen is a week-long regimen, the kit can include seven reusable injectors 302; if the regimen is a two-week-long regimen (as shown in FIGs. 5A and 5B), the kit can include fourteen needle connectors 102. The needle connectors 102 can be indexed 500, for example, by indicating serially what day the needle connectors 102 are to be used (e.g., Day 1, Day 2, etc., as shown in FIG. 5A). Alternatively, the needle connectors can be indexed 500 by days of the week (e.g., Monday, Tuesday, etc.).

It is not necessary, however, that the needle connectors 102 be indexed 500 for any particular day. One purpose of the presently described systems and methods is to limit the number of times the septum 308 is pierced at any given location. To this end, the kit may limit the number of needle connectors 102 having a proximal end 108 needle placement at any given location so that the septum 308 cannot be pierced over a predetermined number of times. It has been shown that piercing a septum 308 over certain number of times can cause deterioration of the rubber material of the septum 308. Depending on the material in which the septum 308 is manufactured, this number can vary. For example, it has been shown that some materials can experience deterioration if the septum 308 is pierced more than five times. In this example, the kit may include no more than five needle connectors 102 having the same proximal end 108 needle location.

Referring again to FIG. 5A for illustration, the example shows seven different configurations of proximal end 108 needle placement, and the kit does not include more than two needle connectors 102 having the same proximal end 108 needle placement. In this example the kit does not need to be indexed 500, because the patient could not pierce the septum 308 more than two times at any given location. The illustration above, as will be appreciated, is for explanation purposes and is not intended to be limiting, as other materials may require no more than two, three, four, six, or more needle connectors 102 having the same proximal end 108 needle placement.

FIG. 5B depicts an end view of a system that includes fourteen needle connectors 102 separated into seven different sets of needle connectors 102, each set having two needle connectors 102 with the same proximal end 108 needle placement. As shown, one of the proximal end 108 needle placements may be positioned in the center of the casing 103, corresponding to the center of the septum 308. If the casing 103 and reusable injector 302 have circular cross sections, this could mean that the proximal end 108 needle placements are concentric with the distal end 304 of the reusable injector. Other proximal end 108 needle placements can be offset at different locations within the casing 103 (i.e., non-concentrically, if circular). For example, if the needle connector 102 includes an anti-rotation feature 110, any proximal end 108 needle placement may be offset from the anti-rotation feature 110 at a radial offset 502. In addition, a first set of needle connectors 102 (e.g., the set for Day 5&6 in FIG. 5B) can be offset by a predetermined radial offset 502 from a second set of needle connectors (e.g., the set for Day 7&8 in FIG. 5B). Again, FIG. 5A is an end view that indicates the placement of the proximal end 108 of the needle within the casing 103, and the view can also indicate a corresponding piercing location on the septum 308. FIG. 5B is a similar end view and is a schematic showing several locations of proximal end 108 needle placement within the casing 103 and/or septum 308.

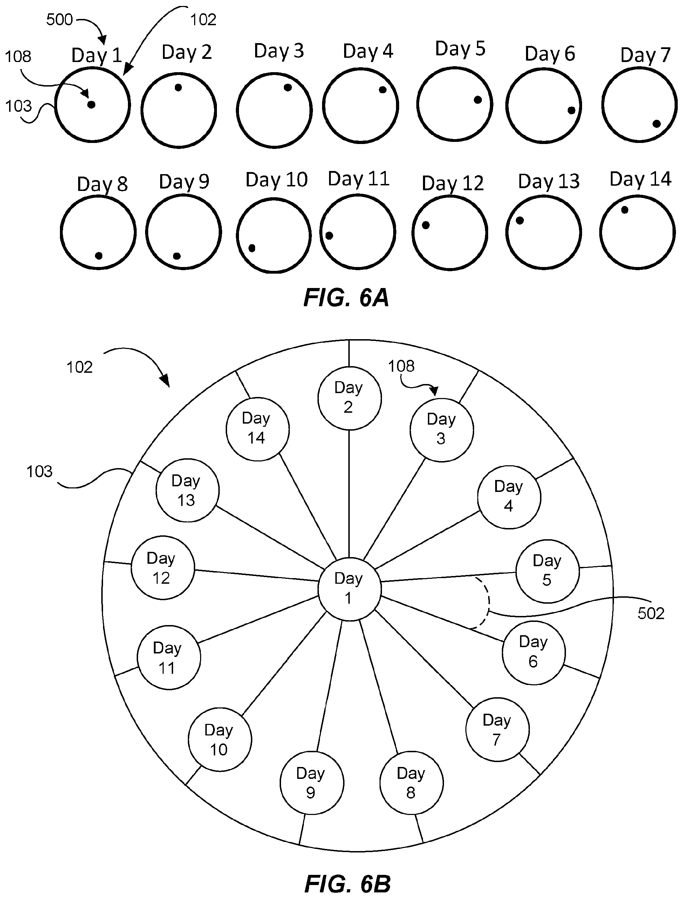

FIGs. 6A and 6B depict an alternative example where the kit includes fourteen different needle connectors 102, and each needle connector 102 has a different proximal end 108 needle placement. In this example, the septum 308 can only be punctured at any one location once. In these cases, the radial offset 502 could smaller than the radial offset 502 described in FIGs. 5A and 5B so as to accommodate the greater number of piercing locations. FIG. 6A is an end view that indicates the placement of the proximal end 108 of the needle within the casing 103, and the view can also indicate a corresponding piercing location on the septum 308. FIG. 6B is a similar end view and is a schematic showing several locations of proximal end 108 needle placement within the casing 103 and/or septum 308.

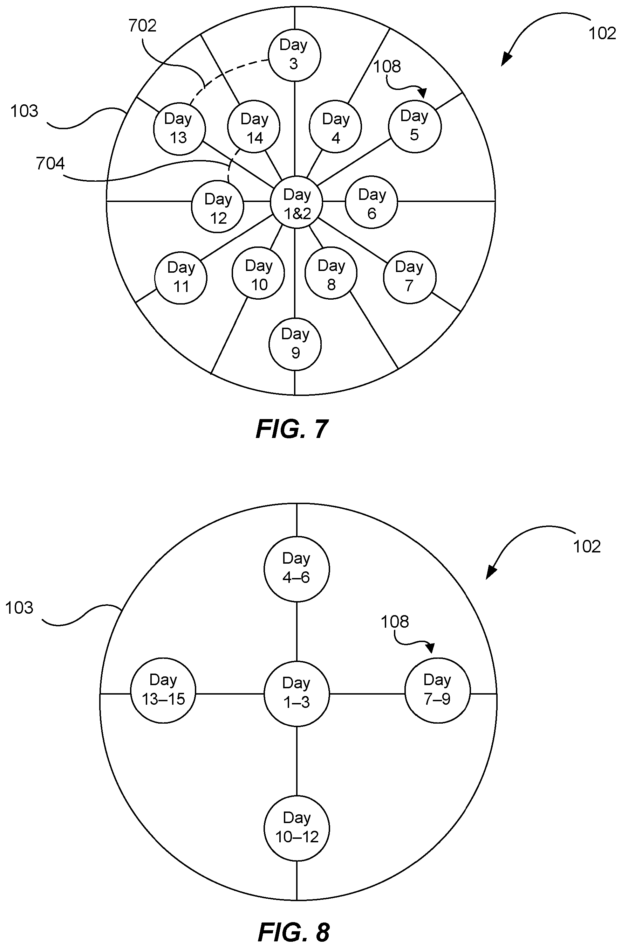

FIG. 7 depicts an alternative proximal end 108 needle placement layout wherein the placements are set radially into an outer ring 702 and an inner ring 704. Depending on the size (i.e., gauge) of the needle 104 with respect to the size (e.g., surface area) of the septum 308, the proximal end 108 needle placements may need to be staggered instead of existing in a single ring (e.g., the placements shown in FIGs. 5A-6B are set into a single ring). For example, in FIG. 6B, if the gauge of the needle 104 was increased slightly, the piercing locations could overlap and cause damage to the material of the septum 308. In FIG. 7, the proximal ends 108 of the needles are placed (a) in the center of the casing 103, (b) radially along an outer ring 702, and (c) radially along an inner ring 704 so as to ensure separate piercing locations are maintained without overlap.

FIG. 8 depicts an example embodiment wherein a set of needle connectors 102 includes more than two connectors having the same proximal end 108 needle placement. As described above, in some examples the septum 308 may not be damaged by inserting the proximal end 108 of the needle 104 into the septum 308 under a certain amount of times. FIG. 8 depicts an example wherein each set includes three needle connectors 102 having the same proximal end 108 needle placement. This number, of course, can vary depending on the needs of the system and the material of the septum 308.

FIG. 9-11 are end views of needle connectors 102 with different cross-sectional geometries. In FIG. 9, the casing 103 is circular except for a flat section, which can be the anti-rotation feature 110 of the needle connector 102 that ensures proper placement of the needle connector 102 upon the distal end 304 of the reusable injector 302. The flat section (i.e., a first anti-rotation feature 110) can correspond to a flat section on the reusable injector 302 (i.e., a second anti-rotation feature 306) to ensure the needle connector 102 is positioned properly so that the proximal ends 108 of the needles 104 piece the septum 308 at the intended location.

FIG. 10 depicts an example system wherein the needle connectors 102 (i.e., the casing 103 of the needle connectors 102) have a square cross section. In this example, the anti-rotation feature 110 of the needle connectors 102 is the cross-sectional geometric shape of the needle connector 102 in combination with the cross-sectional geometric shape of the distal end 304 of the reusable injector 302, which can also be square. FIG. 11 also shows a needle connector 102 design wherein another cross-sectional geometric shape of the casing 103 (e.g., a pentagon) can act as the anti-rotation feature 110 of the needle connectors 102. As described above, the cross-sectional geometric shape of the casing 103 is, of course, not limited to a square or a pentagon, and can include but is not limited to trapezoids, hexagons, and other cross-sectional geometric shapes.

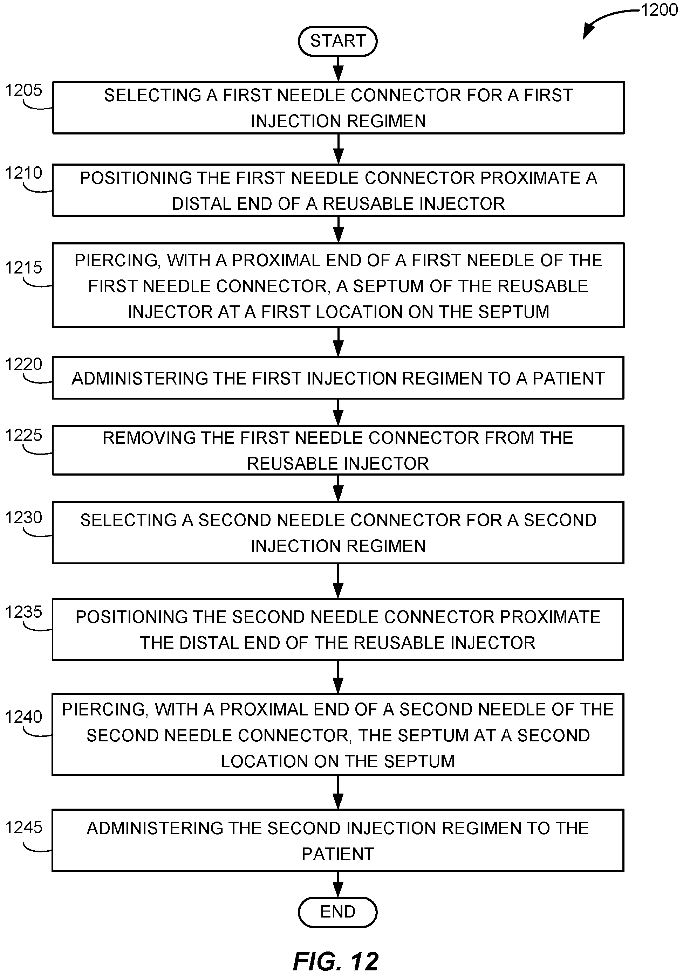

FIG. 12 is a flow diagram illustrating a method 1200 of attaching needle connectors to a reusable injector, according to aspects of the present disclosure. The method steps in FIG. 12 can be implemented by any of the example means described herein or by similar means, as will be appreciated. Referring to method 1200 as outlined in FIG. 12, in step 1205, a first needle connector can be selected for a first injection regimen. The first injection regimen can be associated with a first injection in a multi-injection regimen.

At step 1210, method 1200 can include positioning the first needle connector proximate a distal end of a reusable injector. This step can be similar to the example shown in FIG. 4A above. At step 1215, method 1200 can include piercing, with a proximal end of a first needle of the first needle connector, a septum of the reusable injector at a first location on the septum. To illustrate the step, this piercing of the septum may be completed by the example needle connector shown in FIG. 6B, and the first injection regimen can correspond to "Day 1" (or any other "Day" in the example).

At step 1220, method 1200 can include administering the first injection regimen to a patient. This can be completed, for example, by inserting a distal end of the needle into the skin of the patient. Stated otherwise, the injection regimen can be administered subcutaneously, intravenously, etc. At step 1225, method 1200 can include removing the first needle connector from the reusable injector.

Steps 1230-1245 can be similar to steps 1205-1220 described above but with a different, second needle connector. In particular, at step 1230, method 1200 can include selecting the second needle connector for a second injection regimen. At step 1235, method 1200 can include positioning the second needle connector proximate the distal end of the reusable injector. At step 1240, method 1200 can include piercing, with a proximal end of a second needle of the second needle connector, the septum at a second location on the septum. At step 1245, method 1200 can include administering the second injection regimen to the patient. The first location and the second location are different locations. To illustrate, if the first needle connector described in step 1215 corresponds to the connector "Day 1" in FIG. 6B, the second needle connector can correspond to any other "Day" in FIG. 6B. Method 1200 can end after step 1245. In other embodiments, additional steps according to the examples described above can be performed.

The example needle connectors described above provide a solution for the issue of particle generation by providing a plurality of needle connectors that pierce the septum of a reusable injector at varying locations. Another solution to particle generation includes providing a single needle connector that has varying needle locations so as to vary the puncture site on the septum. The present systems and methods can also provide this solution by employing a single, modular, rotatable needle connector that produces varying needle locations.

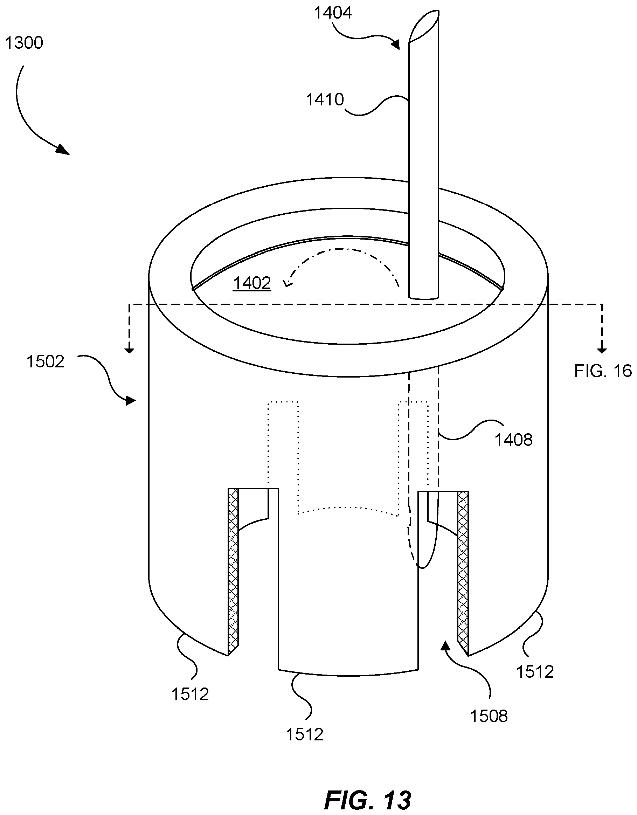

FIG. 13 is a perspective view of a modular needle connector 1300, according to aspects of the present disclosure. The modular needle connector 1300 can include a disk 1402 that is positioned within a connector body 1502. The disk 1402 can rotate within the connector body 1502 so that a needle 1404 within the disk 1402 can move positions with respect to the connector body 1502. As will be described below, this movement of the needle 1404 with respect to the connector body 1502 enables randomized needle placement within a septum 308 of a reusable injector 302, and enables the connector body 1502 to be unscrewed from a reusable injector 302 when the needle 1404 is inside the septum 308. The needle 1404 can remain in a puncture location (and retract axially) as the disk 1402 rotates.

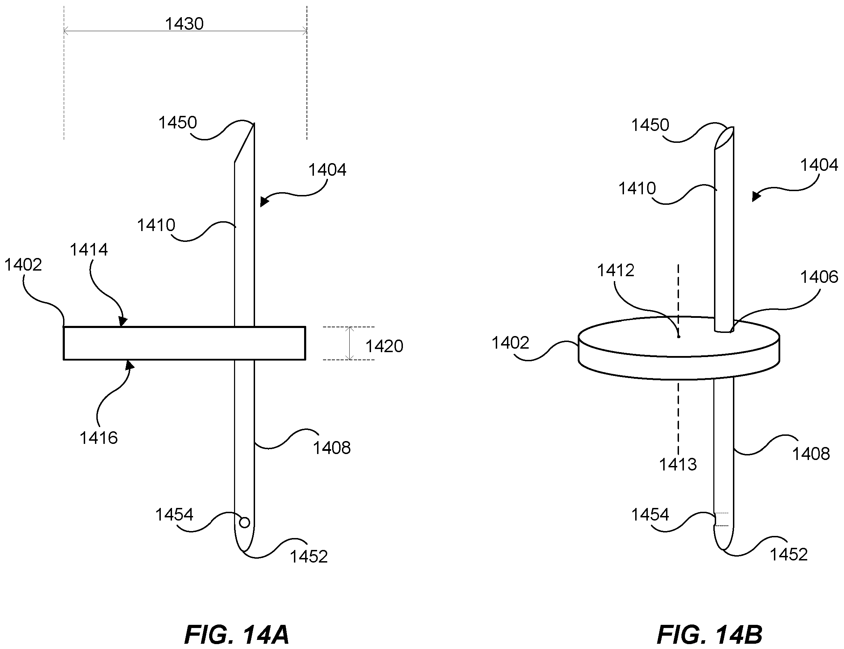

FIGs. 14A and 14B depict and example disk 1402 for a modular needle connector 1300, according to aspects of the present disclosure. FIG. 14A is a side view of the disk 1402; FIG. 14B is a perspective view of the disk 1402. A needle 1404 can be positioned in the disk 1402 at a position off-center from the center 1412. For example, a needle aperture 1406 can be positioned in the disk 1402 at a position that is non-concentric with the center 1412 of the disk 1402. As will be described below, this non-concentric placement of the needle 1404 can facilitate randomized placement of the needle 1404 in the septum 308. The disk 1402 can also include an axis 1413 running though the center 1412 in the case that the disk 1402 is circular. The disk 1402 can be rotatable about the axis 1413. The needle 1404 can be off-axis of the axis 1413 running through the center 1412 of the disk 1402. As will be described in greater detail below, the axis 1413 can also be co-axial with an axis of the septum 308 (e.g., axis 1704).

The needle 1404 can have a proximal end 1408 and a distal end 1410. The distal end 1410 can extend from a first side 1414 of the disk 1402, and the proximal end 1408 can extend from a second side 1416 of the disk 1402. The proximal end 1408 can extend into the connector body 1502, as will be described below, such that it can be inserted into a septum 308 of the reusable injector 302. The proximal end 1408 and the distal end 1410 of the needle 1404 can be a single component extending through the needle aperture 1406, for example a single needle having a tip at both ends. In other examples, the proximal end 1408 and the distal end 1410 of the needle 1404 can be two separate pieces that are joined together proximate the needle aperture 1406.

The tips of both the proximal end 1408 and the distal end 1410 of the needle 1404 can serve different purposes, i.e., piercing a septum and piercing skin, respectively. To this end, the tips of each portion of the needle can be tailored for its particular purpose. For example, a distal tip of the distal end 1410 can include a bevel tip 1450, which is a common type of needle tip for puncturing skin. Of course, any other needle tip can be used for the distal end 1410 of the needle 1404. The proximal end 1408 can be tailored to prevent particle generation via puncturing the septum 308. For example, the tip of the proximal end 1408 can be a non-coring needle tip. Non-coring needle types can include a variety of shapes, including blunt-tipped shapes and needles curved at 45° to prevent a bevel from coring the septum 308. One example tip for the proximal end 1408 is a blunt tip 1452 needle having a side-mounted fluid inlet 1454. Since the fluid inlet 1454 of the needle is placed on the side of the needle instead of at the tip, the chance of coring the septum 308 is decreased while the therapeutic agent can still be drawn into the needle 1404.

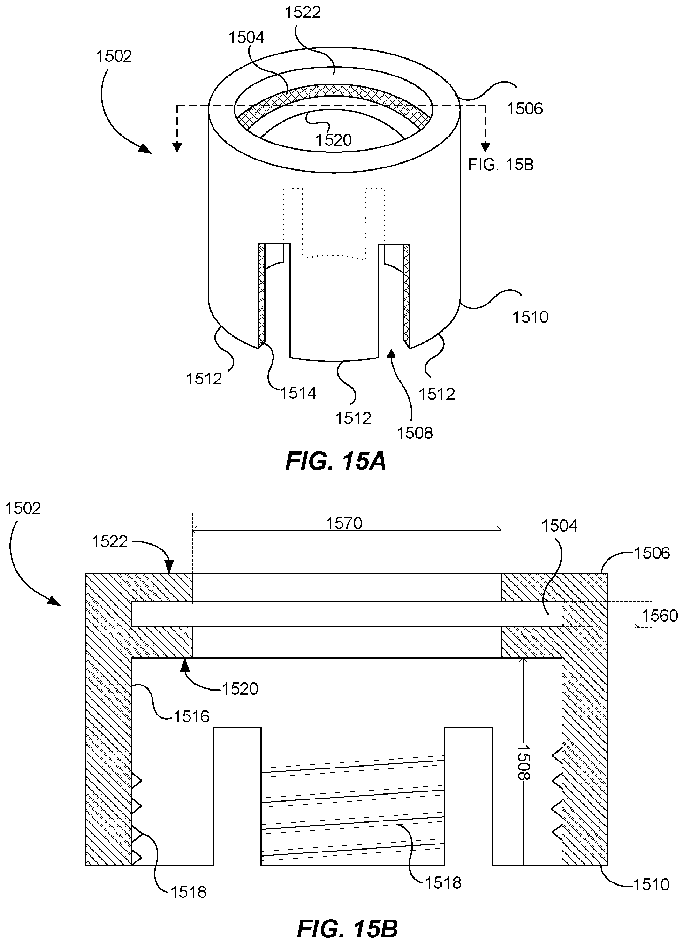

FIGs. 15A and 15B depict and example connector body 1502 for a modular needle connector 1300, according to aspects of the present disclosure. FIG. 15A is a perspective view of the connector body 1502; FIG. 15B is a side sectional view of the connector body 1502. The connector body 1502 can include a disk slot 1504 located near a first end 1506 of the connector body 1502. The disk slot 1504 can be sized to accept the disk 1402. For example, the disk slot 1504 can be circular to accommodate a circular disk 1402. A circular disk 1402 can enable the disk 1402 to rotate within the disk slot 1504. A sufficient tolerance between the size of the disk 1402 and the size of the disk slot 1504 can be provided to further facilitate rotation of the disk 1402. For example, a first height 1420 and first diameter 1430 of the circular disk 1402 can be less than a second height 1560 and a second diameter 1570 of the disk slot 1504 such that the disk 1402 can be rotatable within the disk slot 1504. The disk slot 1504 can be defined at one side by a lower ridge 1520 that faces an injector housing 1508, and at another side by an upper ridge 1522 that faces the first end 1506 of the connector body 1502 (the upper ridge 1522 facing toward the skin during an injection).

The connector body 1502 can define an injector housing 1508 at a second end 1510 of the connector body 1502. The injector housing 1508 can be an internal cavity of the connector body 1502 that accepts the reusable injector 302. Referring to FIG. 15B for illustration, the injector housing can extend from the second end 1510 of the connector body 1502 to the lower ridge 1520 proximate the disk slot 1504. The injector housing 1508 can include features that enable the connector body 1502 to attach to, and be removed from, a reusable injector 302. For example, the connector body 1502 can include wings 1512 extending along at least a portion of a length the injector housing 1508. The wings 1512 can be individual protrusions extending across the injector housing 1508, which can be separated by cutouts 1514 between the wings 1512. The wings 1512 can be flexible such that, when the connector body 1502 is lowered onto the reusable injector 302, the wings flex outward to provide a snapping mechanism onto the distal end 304 of the reusable injector 302. The wings 1512, therefore, enable the connector body 1502 to be lowered axially onto the reusable injector 302 without needing to twist or thread the connector body 1502 onto the injector.

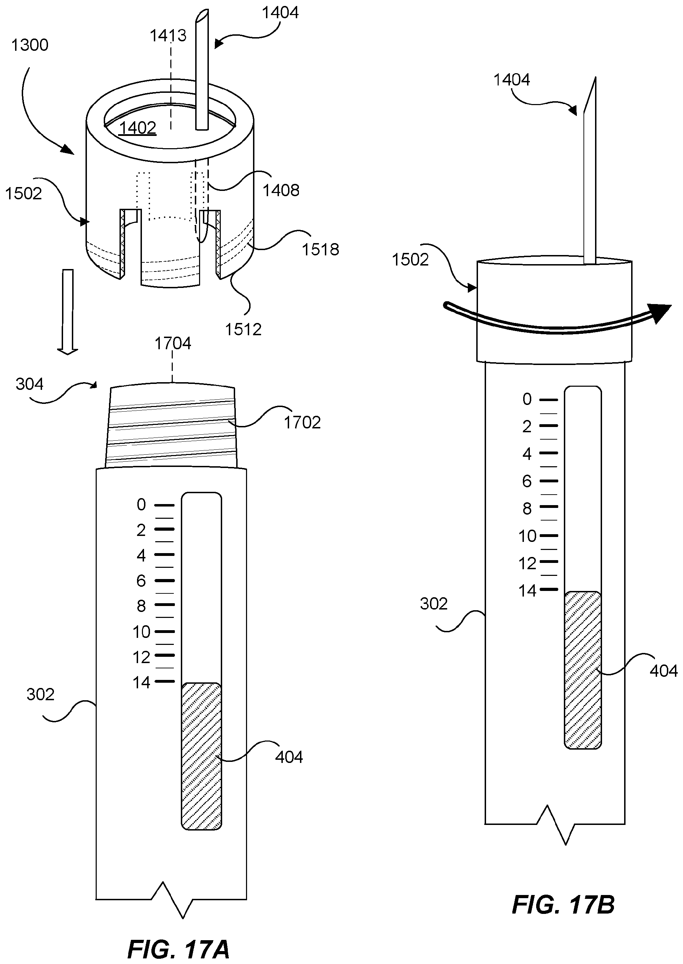

An interior surface 1516 of the injector housing 1508 can also include additional attachment features that enable the connector body 1502 to be securely connected to a reusable injector 302. For example, the interior surface 1516 can include threads 1518 that can be sized to engage threads (e.g., threads 1702 in FIG. 17A) on the distal end 304 of the reusable injector 302. As will be described in greater detail below, the combination of the wings 1512 and the threads 1518 enable different mechanisms for attachment/detachment of the modular needle connector 1300 (e.g., axial pushing for attachment, unscrewing for detachment, etc.).

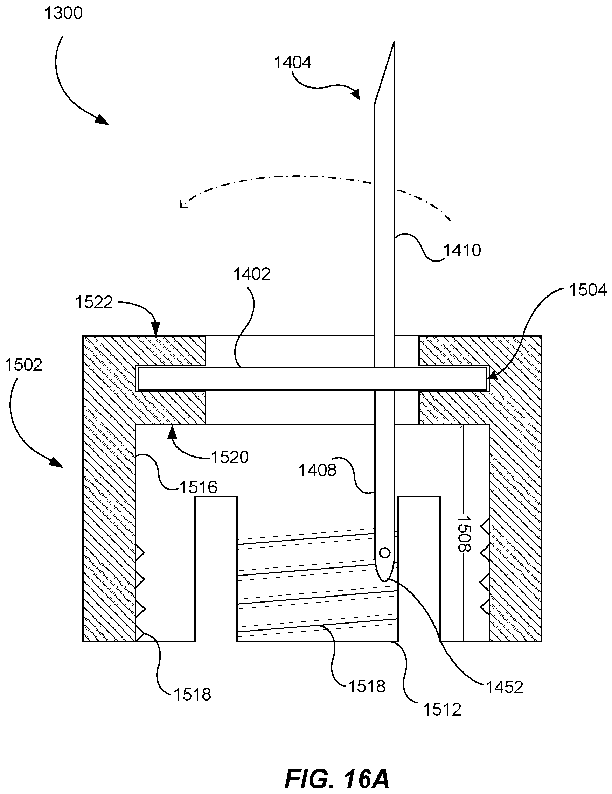

FIG. 16A is a side sectional view of an assembled modular needle connector 1300 (e.g., the modular needle connector shown in FIG. 13), according to aspects of the present disclosure. Once assembled, the disk 1402 can be positioned within the disk slot 1504. The distal end 1410 of the needle 1404 can face externally to the injector housing 1508. The proximal end 1408 of the needle 1404 can face inwardly, i.e., inside of the injector housing 1508. It is contemplated that the disk 1402 can be inserted into the disk slot 1504 in a multistep process. For example, the connector body 1502 can be manufactured in two separate pieces. The disk 1402 can be inserted into the respective disk slot 1504 of each piece, and the two pieces can be glued, heat welded, etc. together to create the construct shown in FIG. 16A. In other examples, one or both of the upper ridge 1522 or the lower ridge 1520 can be ramped such that, with sufficient axial force on the disk 1402, the disk 1402 can be pressed into the disk slot 1504 in one direction, but the disk 1402 is prevented from being removed once inserted.

As described above, the disk 1402 can be rotatable with respect to the connector body 1502. This can enable the variable piercing locations on the septum 308 that were described above with respect to FIGs. 6A-11. Using FIG. 6B for illustration, the embodiments described above for a plurality of needle connectors included different needle connectors 102 having differing needle placements within the casing 103. Accordingly, the septum 308 can be punctured at different locations using different needle connectors 102.

With the module needle connector 1300, a single device can be used to achieve the goal of varying puncture locations. For example, as the disk 1402 freely rotates, the proximal end 1408 of the needle can change position from a first position within the injector housing 1508 to a second position within the injector housing 1508 via rotation of the rotatable disk 1402. This means that, no matter which orientation the user places the modular needle connectors 1300 with respect to the distal end 304 of the reusable injector 302, the placement of the proximal end 1408 of the needle 1404 is random. This means that any of the needle placements shown in FIG. 6A can be achieved by the same device, i.e., the modular needle connector 1300.

The randomized placement of the proximal end 1408 of the needle 1404 can achieve the goal of varying the puncture location on the septum 308. Statistical analysis has shown that the probability of piercing the septum 308 at the same location several times is minimal when the placement of the proximal end 1408 of the needle 1404 with respect to the septum 308 is completely random. Additionally, the number of times a septum 308 can be pierced at the same location before particle generation occurs depends on a number of factors, including the material used for the septum 308 and, particularly, the type of needle being inserted into the septum 308. The randomized placement of a non-coring needle, e.g., the blunt tip 1452 or another non-coring needle, can decrease the occurrence of particle generation in a reusable injector 302.

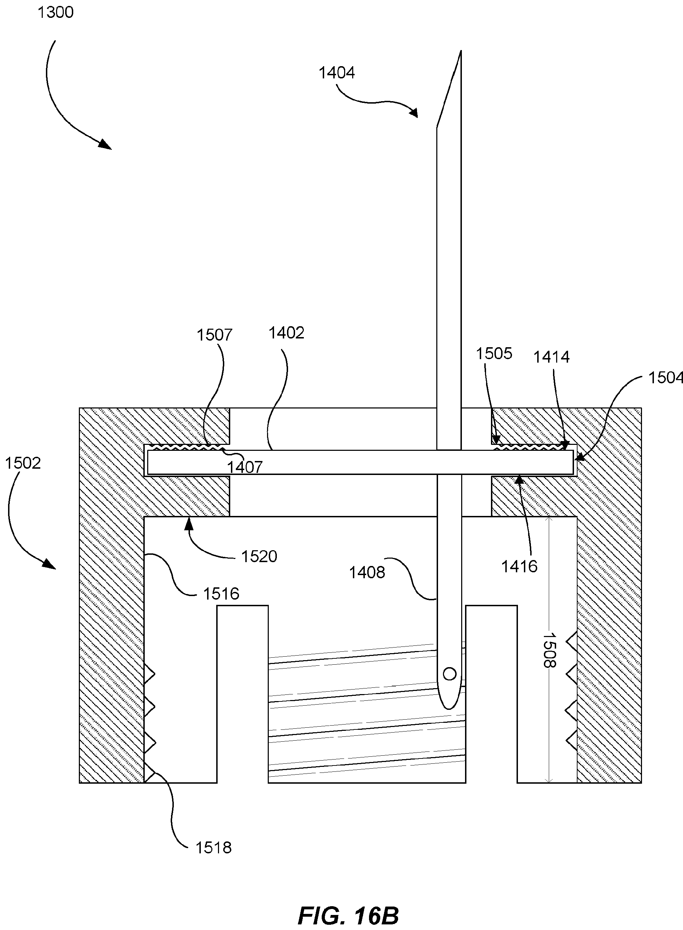

In addition to the example above in FIG. 16A, the first side 1414 of the disk 1402 and the distal side 1505 of the disk slot 1504 can have matching intermeshing teeth 1407, 1507, as shown in FIG. 16B. The fine teeth 1407, 1507 do not engage when the needle 1404 of modular needle connector 1300 is upright. By upright, the second side 1416 rests on the disk slot 1504 and there is a slight gap between the first side 1414 and the distal side 1505 of the disk slot 1504. Once the modular needle connector 1300 is set on the distal end 304 of the reusable injector 302, and the reusable injector 302 inverts for the user to perform an injection, the teeth 1407, 1507 engage. The engagement of the teeth 1407, 1507 add extra friction and prevent any movement of the disk 1402 within the disk slot 1504 during the critical injection period. The teeth 1407, 1507 are extremely fine, to still allow for the almost infinite positioning of the rotating disk 1402. When the reusable injector 302 is again returned to the upright position after injection, the teeth 1407, 1507 disengage and the disk 1402 is again free to rotate during the rotating disengagement of the modular needle connector 1300 from the reusable injector 302. The teeth 1407 can also be positioned on the second side 1416 of the disk 1402 in a similar manner as above.

FIGs. 17A and 17B are side views of an example method of attaching a modular needle connector 1300 to a reusable injector 302, according to aspects of the present disclosure. As shown in FIG. 17A, the modular needle connector 1300 can be positioned near the distal end 304 of a reusable injector 302. A rotatable disk 1402 of the modular needle connector 1300 can have an axis 1413 running through a center 1412 of the disk 1402. The axis 1413 of the disk 1402 can be co-axial with an axis 1704 running through the septum 308 (e.g., when the septum 308 is circular, for example as shown in FIG. 3A). A needle 1404 of the modular needle connector 1300 can, however, be off-axis from the axis 1413 of the disk 1402 and the axis 1704 of the septum 308.

The modular needle connector 1300 can be advanced axially onto the distal end 304 until the proximal end 1408 of the needle 1404 contacts the septum 308 of the reusable injector 302. The modular needle connector 1300 can then be further advanced, such that the proximal end 1408 of the needle 1404 advances through the septum 308 as the modular needle connector 1300 is seated. At a certain point in assembly of the device, the first threads 1518 of the connector body 1502 can contact the second threads 1702 on the distal end 304 of the reusable injector 302.

At this point, continued axial pressure on the modular needle connector 1300 can cause the wings 1512 on the connector body 1502 to bend, or deflect, outward such that the contact between the first threads 1518 and the second threads 1702 can be overcome, and the modular needle connector 1300 can be fully seated. Full seating of the modular needle connector 1300 can occur when the distal end 304 of the reusable injector 302 contacts the lower ridge 1520 of the connector body 1502 or when the second end 1510 of the connector body 1502 meets with a surface of the distal end 304 of the reusable injector 302, for example. The force required to overcome the contact of the first threads 1518 and the second threads 1702 can be modified by the quantity of wings 1512 on the connector body 1502. For example, the connector body 1502 can include four wings 1512, as shown in FIG. 17A. In other examples, a fewer quantity of wings 1512 can be formed into the connector body 1502. If the material from which the connector body 1502 is manufactured is sufficiently pliable/flexible, two wings 1512 can be sufficient to enable the connector body 1502 to be seated over the threads. If the material from which the connector body 1502 is manufactured is more ridged, more than four wings 1512 can be formed into the connector body 1502, e.g., five or more wings.

FIG. 17B depicts a modular needle connector 1300 that is fully seated onto the reusable injector 302. At this point, the reusable injector 302 can be used to administer the therapeutic agent to the patient. Once the injection is complete, the modular needle connector 1300 can be removed from the reusable injector 302. This can be accomplished by unscrewing the modular needle connector 1300 from the reusable injector 302. For example, the first threads 1518 of the connector body 1502 can be disengaged from the second threads 1702 of the reusable injector 302.

Upon rotating the connector body 1502 with respect to the reusable injector 302, the disk 1402 can rotate with respect to the connector body 1502. Stated otherwise, the needle 1404 placement within the septum 308 can remain constant, and, as the connector body 1502 is unscrewed, the proximal end 1408 of the needle 1404 is pulled axially from the puncture hole in the septum 308, facilitated by the rotation of the disk 1402. The modular needle connector 1300 can then be removed from the reusable injector 302.

The process shown in FIGs. 17A and 17B can be repeated for a subsequent dosage of the therapeutic agent using the same modular needle connector 1300. For the subsequent dosage, the modular needle connector 1300 can be positioned near the distal end 304 of a reusable injector 302. The modular needle connector 1300 can then be advanced axially onto the distal end 304 until the proximal end 1408 of the needle 1404 contacts the septum 308 of the reusable injector 302. Since the placement of the proximal end 1408 of the needle 1404 is random, due to the rotatability of the disk 1402, the location of the proximal end 1408 of the needle 1404 will be random and, therefore, likely at a different location that the previous dosage. This process can be repeated for as many doses as are contained in the reusable injector 302, e.g., 14 times for a 14-day regimen. Each of those 14 assemblies have a random needle placement with respect to the septum 308.

It should be noted that the process described with respect to FIGs. 17A and 17B includes a two-step assembly/disassembly process. For assembly, the modular needle connector 1300 can be advanced axially, and the wings 1512 can facilitate the advancement over the threads (e.g., first threads 1518 and second threads 1702). For disassembly, the modular needle connector 1300 can be unscrewed. The present disclosure is not limited to this series, however. For example, the device can be assembled by advancing the modular needle connector 1300 until the proximal end 1408 of the needle 1404 contacts the septum 308, and then the first threads 1518 can engage the second threads 1702. The modular needle connector 1300 can then be fully seated by screwing the modular needle connector 1300 onto the reusable injector 302.

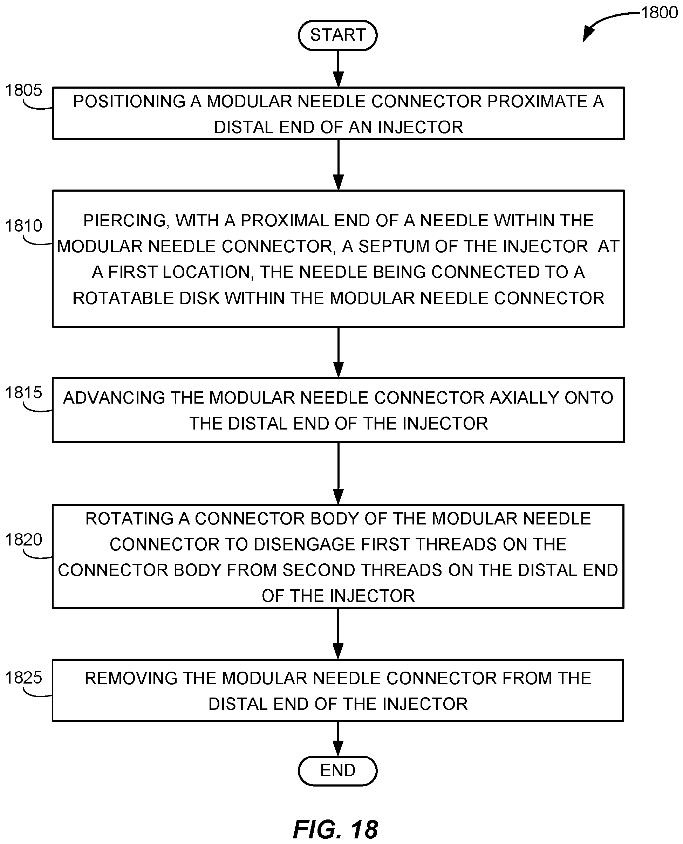

FIG. 18 is a flow diagram illustrating a method 1800 of attaching a modular needle connector with an injector, according to aspects of the present disclosure. The method steps in FIG. 18 can be implemented by any of the example means described herein or by similar means, as will be appreciated. Referring to method 1800 as outlined in FIG. 18, in step 1805, a modular needle connector can be positioned proximate a distal end of an injector. Step 1810 can include piercing a septum of the injector at a first location. The proximal end of a needle within the modular needle connector can pierce the septum. The needle can be connected to a rotatable disk within the modular needle connector.

Step 1815 can include advancing the modular needle connector axially onto the distal end of the injector. At this step, the proximal end of the needle can advance into the septum. The modular needle connector can be advanced until the connector is seated onto the distal end of the injector.

Step 1820 can include rotating a connector body of the modular needle connector to disengage first threads on the connector body from second threads on the distal end of the injector. As the connector body rotates, the rotatable disk can rotate with respect to a connector body and, therefore, stay stationary with respect to the reusable injector. The needle can, therefore, withdraw axially from the septum as the connector body rotates. Step 1825 can include removing the modular needle connector from the distal end of the injector.

Method 1800 can end after step 1825. In other examples, additional steps according to the examples described above can be performed. For example, the process can be repeated by repositioning the needle the modular needle connector proximate the distal end of the injector and piercing, with the proximal end of the needle, the septum at a second location. The second location can be different than the first location in method 1800 because, as described throughout this disclosure, the disk is rotatable and, therefore, placement of the needle on the septum can be random. Additionally, the step of administering therapeutic agent via a distal end of the needle can be performed, for example between steps 1815 and 1820 above.

The descriptions contained herein are examples of embodiments of the disclosure and are not intended in any way to limit the scope of the disclosure. As described herein, the disclosure contemplates many variations and modifications of the aspiration device including using alternative geometries of structural elements, combining shapes and structural elements from various example embodiments, using alternative materials, etc. These modifications would be apparent to those having ordinary skill in the art to which this disclosure relates and are intended to be within the scope of the claims which follow.

## Claims

1. A modular needle connector (1300) comprising:
a circular disk (1402) comprising:
a needle aperture positioned non-concentrically with a center of the circular disk; and
a needle (1404) connected to the disk and extending through the needle aperture, the needle having a distal end extending from a first side of the circular disk and a proximal end extending from a second side of the circular disk; and
a connector body (1502) comprising:
a disk slot (1504) sized to contain the circular disk and positioned at a first end of the connector body; and
an injector housing (1508) positioned at a second end of the connector body engageable with a reusable injector,
wherein the proximal end of the needle is positioned within the injector housing, and further wherein the circular disk is rotatable within the connector body.

2. The modular needle connector of claim 1, wherein a first height and first diameter of the circular disk is less than a second height and a second diameter of the disk slot such that the circular disk is rotatable within the disk slot when the connector body is disengaged with the reusable injector.

3. The modular needle connector of claim 1, wherein the connector body further comprises a plurality of flexible wings (1512) extending along at least a portion of a length the injector housing, optionally wherein an interior surface of the plurality of flexible wings comprises threads (1518) engageable with the reusable injector.

4. The modular needle connector of claim 1, wherein the distal end of the needle comprises a bevel-tip and the proximal end of the needle comprises a non-coring tip, optionally wherein the non-coring tip comprises a blunt tip and a side-mounted fluid inlet.

5. A system comprising:
a reusable injector (302) comprising a septum; and
a modular needle connector according to claim 1,
wherein the proximal end of the needle is movable from a first position within the injector housing to a second position within the injector housing via rotation of the rotatable circular disk.

6. The system of claim 5, wherein the connector body further comprises a plurality of flexible wings engageable with the reusable injector proximate the septum, optionally wherein an interior surface of the plurality of flexible wings comprises first threads engageable with second threads on the reusable injector proximate the septum.

7. The system of claim 5, wherein an interior surface of the injector housing comprises first threads engageable with second threads on the reusable injector proximate the septum.

8. The system of claim 5, wherein the distal end of the needle comprises a bevel-tip and the proximal end of the needle comprises a non-coring tip, optionally wherein the non-coring tip comprises a blunt tip and a side-mounted fluid inlet.

9. The system of claim 5, wherein a first axis of the rotatable disk is co-axial with a second axis of the septum, and the needle is off-axis from the first axis and the second axis.

10. The system of claim 5, wherein the proximal end of the needle is stationary when inserted into the septum, and the rotatable disk is stationary when the connector body is seated onto the reusable injector.

11. A method comprising:
positioning a modular needle connector (1300) proximate a distal end of an injector; (302) piercing, with a proximal end of a needle (1404) within the modular needle connector, a septum of the injector at a first location, the needle being connected to a rotatable disk (1402) within the modular needle connector;
advancing the modular needle connector axially onto the distal end of the injector;
rotating a connector body of the modular needle connector to disengage first threads on the connector body from second threads on the distal end of the injector, wherein rotating the connector body enables the proximal end of the needle to withdraw axially from the first location; and
removing the modular needle connector from the distal end of the injector.

12. The method of claim 11, further comprising:
piercing, with the proximal end of the needle, the septum at a second location; and
rotating the connector body to disengage the first threads from the second threads on the distal end of the injector, wherein rotating the connector body causes the rotatable disk to rotate as the proximal end of the needle is withdrawn axially from the second location.

13. The method of claim 11, wherein the needle is positioned non-concentrically with a center of the rotatable disk.

14. The method of claim 11, wherein advancing the modular needle connector axially onto the distal end of the injector comprises advancing flexible wings over the distal end of the injector, optionally wherein the first threads are positioned on an interior surface of the flexible wings.

## Patentansprüche

1. Modularer Nadelverbinder (1300), umfassend:
eine kreisförmige Scheibe (1402), umfassend:
eine Nadelöffnung, die nicht konzentrisch zu einem Mittelpunkt der kreisförmigen Scheibe positioniert ist; und
eine Nadel (1404), die mit der Scheibe verbunden ist und sich durch die Nadelöffnung erstreckt, wobei die Nadel ein distales Ende, das sich von einer ersten Seite der kreisförmigen Scheibe erstreckt, und ein proximales Ende, das sich von einer zweiten Seite der kreisförmigen Scheibe erstreckt, aufweist; und
einen Verbinderkörper (1502), umfassend:
einen Scheibenschlitz (1504), der bemessen ist, um die kreisförmige Scheibe aufzunehmen, und an einem ersten Ende des Verbinderkörpers positioniert ist; und
ein Injektorgehäuse (1508), das an einem zweiten Ende des Verbinderkörpers positioniert ist und mit einem wiederverwendbaren Injektor in Eingriff bringbar ist,
wobei das proximale Ende der Nadel innerhalb des Injektorgehäuses positioniert ist und wobei ferner die kreisförmige Scheibe innerhalb des Verbinderkörpers drehbar ist.

2. Modularer Nadelverbinder nach Anspruch 1, wobei eine erste Höhe und ein erster Durchmesser der kreisförmigen Scheibe kleiner sind als eine zweite Höhe und ein zweiter Durchmesser des Scheibenschlitzes, sodass die kreisförmige Scheibe innerhalb des Scheibenschlitzes drehbar ist, wenn der Verbinderkörper außer Eingriff mit dem wiederverwendbaren Injektor ist.

3. Modularer Nadelverbinder nach Anspruch 1, wobei der Verbinderkörper ferner eine Vielzahl von flexiblen Flügeln (1512) umfasst, die sich entlang zumindest eines Teils einer Länge des Injektorgehäuses erstreckt, wobei optional eine Innenfläche der Vielzahl von flexiblen Flügeln Gewinde (1518) umfasst, die mit dem wiederverwendbaren Injektor in Eingriff bringbar sind.

4. Modularer Nadelverbinder nach Anspruch 1, wobei das distale Ende der Nadel eine abgeschrägte Spitze umfasst und das proximale Ende der Nadel eine nicht stanzende Spitze umfasst, wobei optional die nicht stanzende Spitze eine stumpfe Spitze und einen seitlich angebrachten Fluideinlass umfasst.

5. System, umfassend:
einen wiederverwendbaren Injektor (302), umfassend ein Septum; und
einen modularen Nadelverbinder nach Anspruch 1,
wobei das proximale Ende der Nadel über eine Drehung der drehbaren kreisförmigen Scheibe von einer ersten Position innerhalb des Injektorgehäuses in eine zweite Position innerhalb des Injektorgehäuses bewegbar ist.

6. System nach Anspruch 5, wobei der Verbinderkörper ferner eine Vielzahl von flexiblen Flügeln umfasst, die nahe dem Septum mit dem wiederverwendbaren Injektor in Eingriff bringbar ist, wobei optional eine Innenfläche der Vielzahl von flexiblen Flügeln erste Gewinde umfasst, die mit zweiten Gewinden an dem wiederverwendbaren Injektor nahe dem Septum in Eingriff bringbar sind.

7. System nach Anspruch 5, wobei eine Innenfläche des Injektorgehäuses erste Gewinde umfasst, die mit zweiten Gewinden an dem wiederverwendbaren Injektor nahe dem Septum in Eingriff bringbar sind.

8. System nach Anspruch 5, wobei das distale Ende der Nadel eine abgeschrägte Spitze umfasst und das proximale Ende der Nadel eine nicht stanzende Spitze umfasst, wobei optional die nicht stanzende Spitze eine stumpfe Spitze und einen seitlich angebrachten Fluideinlass umfasst.

9. System nach Anspruch 5, wobei eine erste Achse der drehbaren Scheibe koaxial zu einer zweiten Achse des Septums ist und die Nadel achsenversetzt zu der ersten Achse und der zweiten Achse ist.

10. System nach Anspruch 5, wobei das proximale Ende der Nadel stationär ist, wenn es in das Septum eingeführt ist, und die drehbare Scheibe stationär ist, wenn der Verbinderkörper auf den wiederverwendbaren Injektor aufgesetzt ist.

11. Verfahren, umfassend:
Positionieren eines modularen Nadelverbinders | (1300) nahe einem distalen Ende eines Injektors (302); Durchstechen eines Septums des Injektors an einem ersten Ort mit einem proximalen Ende einer Nadel (1404) innerhalb des modularen Nadelverbinders, wobei die Nadel mit einer drehbaren Scheibe (1402) innerhalb des modularen Nadelverbinders verbunden ist;
axiales Vorschieben des modularen Nadelverbinders auf das distale Ende des Injektors;
Drehen eines Verbinderkörpers des modularen Nadelverbinders, um erste Gewinde an dem Verbinderkörper außer Eingriff mit zweiten Gewinden an dem distalen Ende des Injektors zu bringen, wobei das Drehen des Verbinderkörpers ermöglicht, dass sich das proximale Ende der Nadel axial von dem ersten Ort zurückzieht; und
Entfernen des modularen Nadelverbinders von dem distalen Ende des Injektors.

12. Verfahren nach Anspruch 11, ferner umfassend:
Durchstechen des Septums an einem zweiten Ort mit dem proximalen Ende der Nadel; und
Drehen des Verbinderkörpers, um die ersten Gewinde außer Eingriff mit den zweiten Gewinden an dem distalen Ende des Injektors zu bringen, wobei das Drehen des Verbinderkörpers bewirkt, dass sich die drehbare Scheibe dreht, während das proximale Ende der Nadel axial von dem zweiten Ort zurückgezogen wird.

13. Verfahren nach Anspruch 11, wobei die Nadel nicht konzentrisch zu einem Mittelpunkt der drehbaren Scheibe positioniert ist.

14. Verfahren nach Anspruch 11, wobei das axiale Vorschieben des modularen Nadelverbinders auf das distale Ende des Injektors das Vorschieben flexibler Flügel über das distale Ende des Injektors umfasst, wobei optional die ersten Gewinde an einer Innenfläche der flexiblen Flügel positioniert sind.

## Revendications

1. Connecteur d'aiguille modulaire (1300) comprenant :
un disque circulaire (1402) comprenant :
une ouverture d'aiguille positionnée de manière non concentrique par rapport à un centre du disque circulaire ; et
une aiguille (1404) reliée au disque et se prolongeant à travers l'orifice de l'aiguille, l'aiguille ayant une extrémité distale se prolongeant d'un premier côté du disque circulaire et une extrémité proximale se prolongeant d'un second côté du disque circulaire ; et
un corps de connecteur (1502) comprenant :
une fente de disque (1504) dimensionnée pour contenir le disque circulaire et positionnée au niveau d'une première extrémité du corps de connecteur ; et
un boîtier d'injecteur (1508) positionné à une seconde extrémité du corps de connecteur pouvant être en prise avec un injecteur réutilisable,
dans lequel l'extrémité proximale de l'aiguille est positionnée à l'intérieur du boîtier d'injecteur, et également dans lequel le disque circulaire peut tourner à l'intérieur du corps de connecteur.

2. Connecteur d'aiguille modulaire selon la revendication 1, dans lequel une première hauteur et un premier diamètre du disque circulaire sont inférieurs à une seconde hauteur et un second diamètre de la fente de disque de sorte que le disque circulaire peut tourner dans la fente de disque lorsque le corps de connecteur n'est plus en prise avec l'injecteur réutilisable.

3. Connecteur d'aiguille modulaire selon la revendication 1, dans lequel le corps de connecteur comprend également une pluralité d'ailes flexibles (1512) se prolongeant le long d'au moins une partie d'une longueur du boîtier d'injecteur, éventuellement dans lequel une surface intérieure de la pluralité d'ailes flexibles comprend des filets (1518) pouvant entrer en prise avec l'injecteur réutilisable.

4. Connecteur d'aiguille modulaire selon la revendication 1, dans lequel l'extrémité distale de l'aiguille comprend une pointe biseautée et l'extrémité proximale de l'aiguille comprend une pointe non carottante, éventuellement dans lequel la pointe non carottante comprend une pointe émoussée et une entrée de fluide montée latéralement.

5. Système comprenant :
un injecteur réutilisable (302) comprenant un septum ; et
un connecteur d'aiguille modulaire selon la revendication 1,
dans lequel l'extrémité proximale de l'aiguille est mobile d'un premier endroit à l'intérieur du boîtier d'injecteur à un second endroit à l'intérieur du boîtier d'injecteur par le biais de la rotation du disque circulaire rotatif.

6. Système selon la revendication 5, dans lequel le corps de connecteur comprend également une pluralité d'ailes flexibles pouvant entrer en prise avec l'injecteur réutilisable près du septum, éventuellement dans lequel une surface intérieure de la pluralité d'ailes flexibles comprend de premiers filets pouvant entrer en prise avec de seconds filets sur l'injecteur réutilisable près du septum.

7. Système selon la revendication 5, dans lequel une surface intérieure du boîtier d'injecteur comprend de premiers filets pouvant entrer en prise avec de seconds filets sur l'injecteur réutilisable près du septum.

8. Système selon la revendication 5, dans lequel l'extrémité distale de l'aiguille comprend une pointe biseautée et l'extrémité proximale de l'aiguille comprend une pointe non carottante, éventuellement dans lequel la pointe non carottante comprend une pointe émoussée et une entrée de fluide montée latéralement.

9. Système selon la revendication 5, dans lequel un premier axe du disque rotatif est coaxial avec un second axe du septum, et l'aiguille est hors axe par rapport au premier axe et au second axe.

10. Système selon la revendication 5, dans lequel l'extrémité proximale de l'aiguille est immobile lorsqu'elle est insérée dans le septum, et le disque rotatif est immobile lorsque le corps de connecteur est placé sur l'injecteur réutilisable.

11. Procédé comprenant :
le positionnement d'un connecteur d'aiguille modulaire | (1300) près d'une extrémité distale d'un injecteur ; (302) le perçage, avec une extrémité proximale d'une aiguille (1404) à l'intérieur du connecteur d'aiguille modulaire, d'un septum de l'injecteur à un premier endroit, l'aiguille étant connectée à un disque rotatif (1402) à l'intérieur du connecteur d'aiguille modulaire ;
l'avancement du connecteur d'aiguille modulaire axialement sur l'extrémité distale de l'injecteur ;
la rotation d'un corps de connecteur de l'aiguille modulaire pour mettre hors prise les premiers filets du corps de connecteur des seconds filets sur l'extrémité distale de l'injecteur, dans lequel la rotation du corps de connecteur permet à l'extrémité proximale de l'aiguille de se retirer axialement du premier endroit ; et
le retrait du connecteur d'aiguille modulaire de l'extrémité distale de l'injecteur.

12. Procédé selon la seconde 11, comprenant également :
le perçage, avec l'extrémité proximale de l'aiguille, du septum à un second endroit ; et
la rotation du corps de connecteur pour mettre hors prise les premiers filets des seconds filets sur l'extrémité distale de l'injecteur, dans lequel la rotation du corps de connecteur entraîne la rotation du disque rotatif lorsque l'extrémité proximale de l'aiguille est retirée axialement du second endroit.

13. Procédé selon la revendication 11, dans lequel l'aiguille est positionnée de manière non concentrique par rapport à un centre du disque rotatif.

14. Procédé selon la revendication 11, dans lequel l'avancement axial du connecteur d'aiguille modulaire sur l'extrémité distale de l'injecteur comprend l'avancement d'ailes flexibles sur l'extrémité distale de l'injecteur, éventuellement dans lequel les premiers filets sont positionnés sur une surface intérieure des ailes flexibles.
